# FASCICULE DE BREVET EUROPEEN

(11) **EP 1 200 458 B1**
(45) Date de publication et mention de la délivrance du brevet: **14.02.2007**
(21) Numéro de dépôt: 00958641.3
(22) Date de dépôt: 28.07.2000
(51) Int. Cl.: C07K 7/00, C07K 14/47, A61P 25/28

(54) **APPLICATIONS DE PEPTIDES ISSUS DU DOMAINE CYTOPLASMIQUE DU PRECURSEUR DE LA PROTEINE AMYLOIDE (APP)**
ANWENDUNGEN VON PEPTIDEN AUS DER ZYTOPLASMISCHEN DOMÄNE DES AMYLOID VORLÄUFERPROTEINS (APP)
USES OF PEPTIDES DERIVED FROM THE CYTOPLASMIC DOMAIN OF THE AMYLOID PROTEIN PRECURSOR (APP)

(30) Priorité: 30.07.1999 FR 9909929
(43) Date de publication de la demande: 02.05.2002
(73) Titulaire: CENTRE NATIONAL DE LA RECHERCHE SCIENTIFIQUE (CNRS), 75794 Paris Cedex 16 (FR)
(72) Inventeur: ALLINQUANT, Bernadette, F-75013 Paris (FR); PROCHIANTZ, Alain, F-75006 Paris (FR)
(74) Mandataire: Orès, Bernard
(86) Numéro de dépôt international: PCT/FR2000/002174
(87) Numéro de publication internationale: WO 2001/009170

(56) Documents cités:
- WO-A-93/07296
- WO-A-94/28412
- WO-A-98/28334
- WO-A-98/38861
- US-A- 5 652 092
- US-A- 5 656 477
- US-A- 5 703 209

## Description

La présente invention est relative à de nouvelles applications de peptides issus du domaine cytoplasmique du précurseur de la protéine amyloïde. Le précurseur de la protéine amyloïde (APP pour *Amyloïd Precursor Protein*) est une protéine de fonction inconnue, dont la forme neuronale comporte 695 acides aminés ; elle présente un seul domaine transmembranaire (positions 625-648) et un court domaine cytoplasmique de 47 acides aminés (positions 649-695), représenté dans la liste de séquences en annexe sous le numéro SEQ ID NO:1.

La maladie d'Alzheimer est un désordre neurodégénératif qui affecte de 1 à 6 % de la population âgée de plus de 65 ans. L'une de ses caractéristiques est la présence de plaques séniles qui contiennent du β-amyloïde (βA4 ou BAP), produit toxique dérivé de l'APP et constitué de peptides de 39 à 42 acides aminés, engendrés par clivage de l'APP par deux protéases, la β et la γ sécrétase. Par ailleurs, une troisième enzyme, dite a sécrétase clive l'APP entre les sites β et γ rendant donc impossible la formation du βA4 supposé pathogène. Aucune de ces secrétases n'a été identifiée à ce jour, même si des suspicions légitimes pèsent sur la protéine PS1 (produit du gène Prcscnilin-1, muté dans des formes familiales de la maladie d'Alzheimer). En effet, PS1 pourrait être soit la γ sécrétase soit un de ses co-facteurs. Enfin, d'autres sites de clivage existent dans le domaine C-terminal dont le site des caspases (N. Barnes et al., J. Neuroscience, 1998, 18, 15, 5869-5880), entre les résidus aspartate et alanine de la SEQ 117 NO:1 (positions 16 et 17). Il reste que les mécanismes responsables de la toxicité du βA4 ne sont pas connus et que la relation entre la présence du βA4 dans les plaques et la pathologie n'est pas élucidée. Il est probable que d'autres facteurs et/ou d'autres domaines de la molécule entrent également en jeu.

C'est la raison pour laquelle de nombreuses études ont essayé d'établir le rôle physiologique et/ou physiopathologique de l'APP et des différents produits de son métabolisme. En effet, le ligand physiologique - s'il existe - du domaine N-terminal n'a pas été identifié et les voies de signalisation sont encore mal définies. Une des stratégies d'accès à l'analyse de ces voies de signalisation est l'identification de partenaires moléculaires du domaine cytoplasmique.

Le domaine cytoplasmique de l'APP ainsi que différents peptides issus de ce domaine cytoplasmique ont en particulier été étudiés :
- les séquences YTSI, KKKQYTSIHHGVVEV (SEQ ID NO:8), GYENPTY (SEQ ID NO:9) et NPTY ont été identifiées comme des signaux d'internalisation ; de manière plus précise, elles sont considérées comme des séquences de transcytose de l'APP entre les compartiments basolatéral et apical des cellules épithéliales MDCK (Haass et al., J. Cell Biol., 1995, 128, 4, 537-547 ; Lai et al., J. Biol. Chem., 1995, 270, 8, 3565-3573 ; Lai et al., J. Biol. Chem., 1998, 273, 6, 3732-3739) ;
- le domaine cytoplasmique C-terminal (APP-Cter) a été identifié comme :
   - intervenant dans la régulation de l'activité GTPasique de la sous-unité αo de la protéine G hétérotrimérique (Brouillet et al., J. Neuroscience, 1999, 19, 5, 1717-1727) ;
   - interagissant avec plusieurs protéines : Pat-1 interagit avec le fragment BaSS (basolateral sorting signal) (KKKQYTSIHHG) du domaine juxtamembranaire et avec le domaine C-terminal complet et interviendrait dans le transport d'APP le long des microtubules, vers la surface cellulaire (Zheng et al., PNAS, 1998, 95, 14745-14750) ; la sous-unité αo de la protéine G hétérotrimérique interagit avec la région médiane dudit domaine cytoplasmique C-terminal, au niveau du doublet d'histidines (HH) (Nishimoto et al., Nature, 1993, 362, 75-79) et la protéine Fe65 avec la région la plus distale du domaine APP-Cter (Fiore et al., J. Biol. Chem., 1995, 270, 52, 30853-30856).

Ces différents résultats montrent la complexité des mécanismes dans lesquels le précurseur de la protéine amyloïde (APP) est impliqué.

Le Brevet US 5,652,092 décrit des peptides correspondant à la séquence cytoplasmique APP : colonne 5, lignes 9-23 ; figure 9B ; SEQ ID NO: 10, 14-18 ; toutefois, le but de ce Brevet est de mieux comprendre comment le BAP neurotoxique est généré à partir de l'APP, et de disposer d'outils de criblage de composés qui réduisent la formation de BAP.

Les Inventeurs ont maintenant montré, que de manière surprenante, des peptides comprenant le domaine juxtamembranaire (positions 649-664) du domaine cytoplasmique du précurseur de la protéine-amyloïde (APP), présentent, après internalisation dans des cellules, une activité apoptotique.

La présente invention a pour objet des peptides, caractérisés en ce qu'ils sont constitués par des séquences incluant le domaine juxtamembranaire du domaine cytoplasmique du précurseur de la protéine amyloïde (APP) (code une lettre), sélectionnées dans le groupe constitué par les séquences Y₁KQYTSIHHGY₀ (SEQ ID NO:2), Y₁KKQYTSIHHGY₀ (SEQ ID NO:3) et Y₁KKKQYTSIHHGY₀ (SEQ ID NO:4), dans lesquelles Y₀ est nul ou représente V, VV, VVE VVEV ou VVEVD et Y₁ représente un peptide d'internalisation et d'adressage issu de la 3^{ème} hélice des homéodomaines et de peptides structurellement apparentés et répond de préférence à la séquence X₁X₂X₃X₄X₅X₆X₇X₈X₉X₁₀X₁₁X₁₂X₁₃X₁₄X₁₅X₁₆, dans laquelle X₁, X₂, X₃, X₄, X₅, X₆, X₇, X₈, X₉, X₁₀, X₁₁, X₁₂, X₁₃, X₁₄, X₁₅, X₁₆ représentent chacun un acide α-aminé, 6 à 10 d'entre lesdits acides aminés étant hydrophobes et X₆ représentant un tryptophane.

Parmi les séquences Y₁ préférées, on peut citer la séquence KQIKIWFQNRRMKWKK (SEQ ID NO:5).

Les peptides X₁X₂X₃X₄X₅X₆X₇X₈X₉X1₀X₁₁X₁₂X₁₃X₁₄X₁₅X₁₆ ont notamment été décrits dans la Demande Internationale WO 97/12912.

Les peptides selon l'invention provoquent l'apoptose des cellules dans lesquelles ils sont internalisés et peuvent avantageusement être utilisés pour sélectionner et cribler des produits aptes à inhiber l'apoptose cellulaire.

La présente invention a donc également pour objet l'utilisation d'un peptide comprenant la séquence KKKQYTSIHHGVVEVD (domaine juxtamembranaire du domaine cytoplasmique du précurseur de la protéine amyloïde (APP)), pour la sélection et le criblage de produits aptes à inhiber l'apoptose.

Selon un mode de réalisation avantageux de ladite utilisation, ledit peptide comprenant le domaine juxtamembranaire du domaine cytoplasmique du précurseur de la protéine amyloïde (APP) est associé à un peptide d'internalisation sélectionné dans le groupe constitué par des peptides aptes à passer la barrière hémato-encéphalique.

A titre d'exemples de peptides d'internalisation aptes à être mis en oeuvre dans la présente invention, on peut citer :
- les peptides d'internalisation et d'adressage, issus de la 3^{ème} hélice des homéodomaines et les peptides structurellement apparentés à ces derniers,
- les peptides issus de protéines virales : VP22 (G. Elliott et al., Cell, 1997, 88, 223-233 ; A. Prochiantz, Current Opinion in Cell Biology, 2000, 12, 399-406) ; les peptides issus du domaine de transduction de la protéine Tat du VIH (Schwarze SR et al., Science, 1999, 285, 5433, 1569-1572),
- ainsi que d'autres peptides tels que ceux décrits dans A. Prochiantz, 2000, précité ; M. Lindgren et al., TIPS, 2000, 21, 99-103 ou C. Rousselle et al., Mol. Pharrnacol., 2000, 57, 679-686 (peptides amphiphiles, peptides issus de séquences signal, transportan etc...).

De manière préférée, le peptide utilisé dans la présente invention est sélectionné dans le groupe constitué par les séquences (code une lettre) Y₁KQYTSIHHGY₀ (SEQ ID NO:2), Y₁KKQYTSIHHGY₀ (SEQ ID NO:3) et Y₁KKKQYTSIHHGY₀ (SEQ ID NO:4), dans lesquelles Y₀ est nul ou représente V, VV, VVE VVEV ou VVEVD et Y₁ est nul ou représente un peptide d'internalisation et d'adressage issu de la 3^{ème} hélice des homéodomaines et de peptides structurellement apparentés et répond de préférence à la séquence X₁X₂X₃X₄X₅X₆X₇X₈X₉X₁₀X₁₁X₁₂X₁₃X₁₄X₁₅X₁₆, dans laquelle X₁, X₂, X₃, X₄, X₅, X₆, X₇, X₈, X₉, X₁₀, X₁₁, X₁₂, X₁₃, X₁₄, X₁₅, X₁₆ représentent chacun un acide α-aminé, 6 à 10 d'entre lesdits acides aminés étant hydrophobes et X₆ représentant un tryptophane.

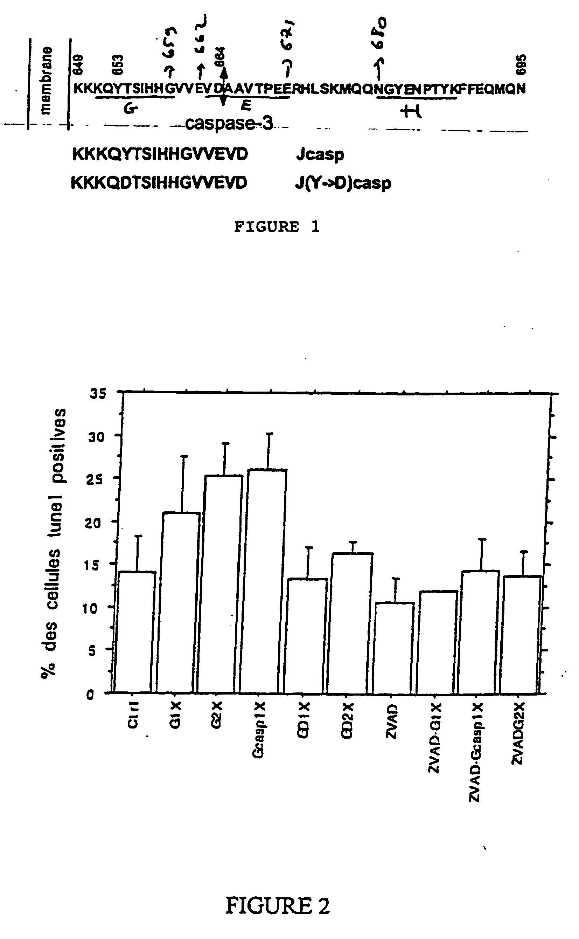

Le peptide de SEQ ID NO:2 dans laquelle Y₁ est nul et Y₀ est nul est dénommé peptide G (voir également figure 1).

Le peptide de SEQ ID NO:4 dans laquelle Y₁ est nul et Y₀ représente VVEVD est dénommé Jcasp (ou Gcasp).

La présente invention a également pour objet l'utilisation de cellules, dans lesquelles un peptide tel que défini ci-dessus a été intemalisé, pour la sélection et le criblage de produits aptes à inhiber l'apoptose.

La présente invention a également pour objet un procédé de criblage et de sélection de produits aptes à inhiber l'apoptose, caractérisé en ce qu'il comprend :
- la mise en contact de l'inhibiteur potentiel avec une cellule dans laquelle un peptide tel que défini ci-dessus a été internalisé et
- la mesure du clivage de l'ADN (révélé notamment par le marquage TUNEL) ou de l'actine (révélé par exemple par un anticorps anti-fractine) ou la mesure de la sous-unité p20 de la caspase 3 (par exemple par marquage spécifique).

Les peptides selon l'invention provoquent l'apoptose des cellules dans lesquelles ils sont internalisés.

La présente invention a donc en outre pour objet l'utilisation d'un peptide tel que défini ci-dessus, pour la préparation d'un médicament anti-cancéreux.

La présente invention a également pour objet des peptides caractérisés en ce qu'ils sont sélectionnés dans le groupe constitué par les séquences (code une lettre) Y₁KQYTSIHHGY₀ (SEQ ID NO:2) et Y₁KKQYTSIHHGY₀ (SEQ ID NO:3), dans lesquelles Y₀ est nul ou représente V, VV, VVE VVEV ou VVEVD et Y₁ est nul, et par le peptide de formule Y₁KKKQYTSIHHGY₀ (SEQ ID NO:4), dans lequel Y₀ représente VVEVD et Y₁ est nul.

L'invention sera mieux comprise à l'aide des figures annexées dans lesquelles :
- la figure 1 représente la séquence du domaine cytoplasmique du précurseur de la protéine amyloïde (APP) : positions 649-695, ainsi que certains de ses fragments : peptide G : positions 651-659 ; peptide E : positions 663-671 ; peptide H : positions 680-688 ; peptide Jcasp : positions 649-664,
- les figures 2 et 3 représentent la quantification du clivage de l'ADN par la technique TUNEL 24 h après internalisation des peptides,
- les figures 4 et 5 représentent la quantification du clivage de l'actine par la caspase 3 à l'aide d'un anticorps anti-fractine,
- la figure 6 illustre la détection de la p20 dans un neurone par immunomarquage (flèche) (phosphatase alcaline) ; la p20 est présente dans tous les compartiments ; échelle : 10 µm
- la figure 7 illustre l'activation de la p20 par le peptide Jcasp (2,4 µM)
- la figure 8 illustre les résultats obtenus *in vivo :* diagrammes représentatifs (une expérience, un animal par condition) de la distribution des cellules positives à la fractine dans des coupes adjacentes. La valeur 0 est arbitrairement attribuée au site d'injection. Le peptide Jcasp montre un plus grand nombre de cellules positives à la fractine comparé au peptide J(Y→D)casp ou au contrôle.

### EXEMPLE 1: Matériel et Méthodes

### 1.1 Cultures neuronales primaires

Des neurones corticaux et corticostriataux sont préparés, comme décrit précédemment (Lafont et al., Development, 1992, 114, 17-29), à partir d'embryons de souris E14 ou d'embryons de rat E15.

Brièvement, les cellules dissociées sont étalées sur des plaques en plastique (plaques de type ELISA) revêtues de polyornithine à une densité de 5 000 cellules par puits et incubées dans un milieu convenable complémenté en hormones, protéines et sels.

Pour vérifier l'internalisation du peptide étudié, les cellules sont étalées sur des lames en verre recouvertes de polyornithine, à une densité de 100 000 cellules par lame.

### 1.2 Préparation des peptides

On utilise le vecteur V1 (Pénétratine ou P = KQIKIWFQNRRM KWKK) (SEQ ID NO:5) comme peptide d'internalisation qui, après fusion génétique ou chimique à un cargo, permet sa translocation à travers la membrane plasmique et son adressage cytoplasmique et nucléaire.

Plusieurs peptides ont ainsi été préparés :
- SEQ ID NO:5 + domaine cytoplasmique entier de l'APP (SEQ ID NO:1).
- **Y₁KKKQYTSIHHGY₀:** SEQ ID NO:4 dans laquelle Y₀ est nul ou représente VVEVD (Jcasp) et Y₁ représente la SEQ ID NO:5 ; la partie en gras correspond au peptide G de la figure 1.
- **Y₁KQYTSIHHGY₀:** SEQ ID NO:2 dans laquelle Y₀ est nul (peptide G) et Y₁ représente la SEQ ID NO:5 ; la partie en gras correspond au peptide G de la figure 1.
- **Y₁KKQYTSIHHGY₀:** SEQ ID NO:3 dans laquelle Y₀ est nul et Y₁ représente la SEQ ID NO:5 ; la partie en gras correspond au peptide G de la figure 1.
- SEQ ID NO:5 + domaine E (VDAAVTPEE, SEQ ID NO:6), souligné dans la séquence selon la figure 1.
- SEQ ID NO:5 + domaine H (NGYENPTYK, SEQ ID NO:7), souligné dans la séquence selon la figure 1.
- SEQ ID NO:5 + peptide correspondant à la séquence MYC [EQKLISEED] (peptide Pmyc).
- SEQ ID NO:5 + peptide J(Y→D)casp.

Le peptide G correspond à un signal de transcytose et comprend un résidu tyrosine (Y), le peptide a également été internalisé soit après phosphorylation de cette tyrosine (Y-P) soit après sa substitution par une alanine (Y→A) ou un aspartate (Y→D). Les deux substitutions abolissent totalement les effets physiologiques de G alors que la phosphorylation les amenuise sans les abolir. Dans la mesure où Y→D -mime-une-phosphorylation on peut proposer comme hypothèse parcimonieuse que la tyrosine est nécessaire, mais que sa phosphorylation ne l'est probablement pas, l'effet intermédiaire de Y-P étant alors explicable par la désphosphorylation du peptide dans la cellule. On ne peut cependant pas exclure que la phosphorylation est nécessaire mais que la substitution Y→D n'est pas suffisante pour la mimer.

Ces différents peptides sont synthétisés chimiquement (pureté 95-98 %, Synthem, France) avec (Jcasp et J(Y→D)casp) ou sans biotine N-terminale et bras espaceur d'acide aminopentanoïque (Derossi et al., J. Biol. Chem., 1994, 269, 10444-10450).

Il y a lieu de noter que dans la mesure où les 2 derniers acides aminés de la séquence SEQ ID NO:5 sont des lysines (KK), le peptide G (KQYTSIHHG) se trouve artificiellement rallongé de 2 acides aminés.

### 1.3 Internalisation des peptides recombinants dans des neurones

Les conditions d'internalisation sont les mêmes que celles décrites dans la Demande internationale WO 97/12912.

Tous les peptides sont ajoutés aux cellules deux heures après l'étalement de ces dernières. L'internalisation est vérifiée par microscopie confocale après immunomarquage (Pmyc) ou détection de la biotine (Jcasp et ses variants).

L'internalisation et la stabilité intracellulaire de Jcasp, Pmyc et J(Y→D)Casp sont identiques. Les inhibiteurs irréversibles de la caspase zVAD-fmk (100 µM) et zDEVD-fmk (200 µM) (Calbiochem, France) sont ajoutés 1 heure avant l'addition du peptide.

### 1.4 Immunocytochimie et quantification des cellules apoptotiques

Les cellules apoptotiques sont détectées par marquage TUNEL (kits à la fluorescéine ou à la phosphatase alcaline) comme décrit par le fournisseur (Roche diagnostics, France).

Pour l'immunodétection de la fractine ou de la sous-unité p20 de la caspase 3 (Pharmingen), les cellules sont fixées au paraformaldéhyde à 4 % (30 minutes, à température ambiante), lavées trois fois avec du PBS et saturées 1 heure à 37°C avec du sérum de veau foetal à 10 % (FCS) dans du PBS contenant 0,2 % de Triton X 100.

Des anticorps primaires purifiés et dirigés contre la fractine ou la p20 sont dilués au 1/2000 et au 1/500 respectivement (dans du PBS-FCS), incubés une nuit à 4°C, lavés trois fois et incubés avec des anticorps anti-lapin biotinylés.

La détection est réalisée à l'aide du kit d'amplification phosphatase alcaline (Vector, France).

Pour chaque condition, 600 à 800 cellules sont comptées trois fois.

L'analyse statistique est réalisée avec ANOVA et test de Scheffé.

### 1.5 Tests in vivo

1 µl (0,2 µl/min.) de 2,7 µM de Jcasp (n=8), J(Y→D)Casp (n=6) ou de PBS (n=3) est injecté stéréotaxiquement dans le cortex de souris adultes aux coordonnées A=0, L=2 et D=1,5 (Atlas du cerveau de souris par KBJ Franklin et G.Paxinos, *Academic Press*). 24 heures après, les animaux sont sacrifiés, perfusés avec du paraformaldéhyde à 4 % et les cerveaux sont extraits et cryoprotégés.

Des coupes congelées (16 µm d'épaisseur) sont préparées et utilisées pour une détection TUNEL ou par immunocytochimie de la fractine, en utilisant l'anticorps primaire purifié (dilution au 1/100^{éme} dans du PBS-FCS) sans amplification et un anticorps secondaire anti-lapin marqué au Cy3 et dilué au 1/400^{éme} (Jackson Immunoresearch Laboratories, Inc.). Le nombre de cellules positives à la fractine est compté sur des coupes adjacentes. L'analyse statistique est réalisée par ANOVA et test de Fischer.

### EXEMPLE 2 : Résultats in vitro

### 2.1. lnduction d'une apoptose neuronale

L'internalisation du domaine C-ter entier (APP-Cter) n'est pas toxique mais a, cependant, un effet négatif sur la croissance neuritique. L'internalisation des peptides E et H est sans effet alors que celle du peptide G, à des concentrations inférieures au µM reproduit les effets du domaine C-terminal intact.

Le résultat le plus intéressant est que le peptide G, à des concentrations de l'ordre de 1 à 1,5 µM ou le peptide Jcasp à des concentrations de 1,2 à 2,4 µM entraîne la mort des neurones et que cette mort correspond à un processus apoptotique, donc régulé.

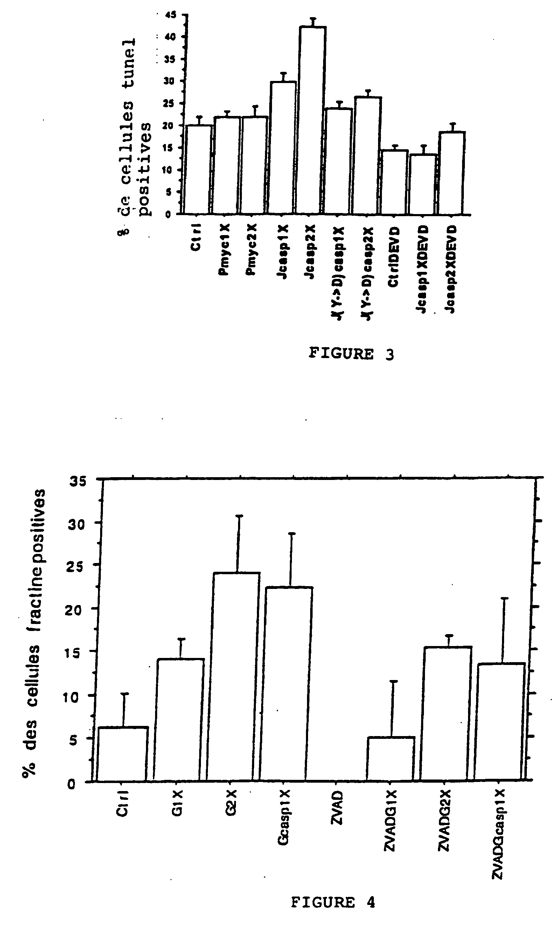

Le caractère apoptotique de la mort provoquée par l'internalisation du peptide G ou du peptide Jcasp (figures 2-7) est démontré par la fragmentation de l'ADN, mise en évidence par la méthode dite « TUNEL » (figures 2 et 3), et par l'activation des caspases (figures 4-7). L'activation des caspases est démontrée par l'apparition de formes clivées de l'actine et par le blocage de l'apoptose par des inhibiteurs de caspase à large spectre d'activité (inhibiteur de caspase 1, 3, 4 et 7) comme le zVAD ou le zDEVD-fink (figures 4, 5 et 7), plus spécifique de la caspase 3.

Les figures 2 et 3 illustrent la quantification du clivage de l'ADN par la technique TUNEL 24h après internalisation des peptides.

Le peptide G a été internalisé à 2 concentrations (1X et 2X) et le peptide Gcasp (ou Jcasp) à la concentration 1X, en présence ou en l'absence de l'inhibiteur de caspases zVAD.

Chaque condition a été testée en triple. Le pourcentage de cellules positives a été évalué après 24h, par comptage d'environ 1000 cellules par puits. Le graphe indique une augmentation significative du clivage de l'ADN en présence du peptide G seul (concentration 1X : p<0.0001 ; concentration 2X : p<0.0001) et du peptide Jcasp (ou Gcasp) (KKKQYTSIHHGVVEVD) (SEQ ID NO:4 dans laquelle Y₀ = VVEVD et Y₁ = SEQ ID NO:5) (concentration 1X : p<0.0001). Le ZVAD inhibe cette augmentation du clivage.

Le peptide Jcasp induit une apoptose neuronale. Deux heures après l'étalement sur plaque, le peptide Jcasp est ajouté aux neurones corticaux de rat E15 et la mort cellulaire est évaluée par effet TUNEL, 24 heures plus tard. La figure 3 montre que le peptide Jcasp (1,2 et 2,4 µM) conduit une fragmentation de l'ADN.

La substitution de la tyrosine par un aspartate diminue, comme pour le peptide G la mort cellulaire tandis que l'internalisation d'un peptide myc sans relation avec APP et lié à la pénétratine (Pmyc) n'a pas d'effet sur le nombre de cellules positives obtenues par la méthode TUNEL.

Dans la mesure où la fragmentation de l'ADN suggère une apoptose, la même expérience a été réalisée en présence de zDEVD-fmk qui inhibe la caspase 3. A 200 µM, zDEVD-fmk a un faible effet sur la mort cellulaire basale et inhibe la fragmentation de l'ADN induite par le peptide Jcasp à 1,2 µM et 2,4 µM (figure 3).

Des inhibitions similaires sont obtenues avec l'inhibiteur zVAD-fmk (100 µM), (voir figure 2).

Le peptide Jcasp non lié à la pénétratine (séquence d'internalisation de SEQ ID-NO:5) qui n'est donc pas internalisé, n'induit pas de fragmentation de l'ADN.

### 2.2. L'induction de l'apoptose est liée à l'activation de la caspase 3

### - quantification du clivage de l'actine par la caspase 3 par un anticorps anti-fractine

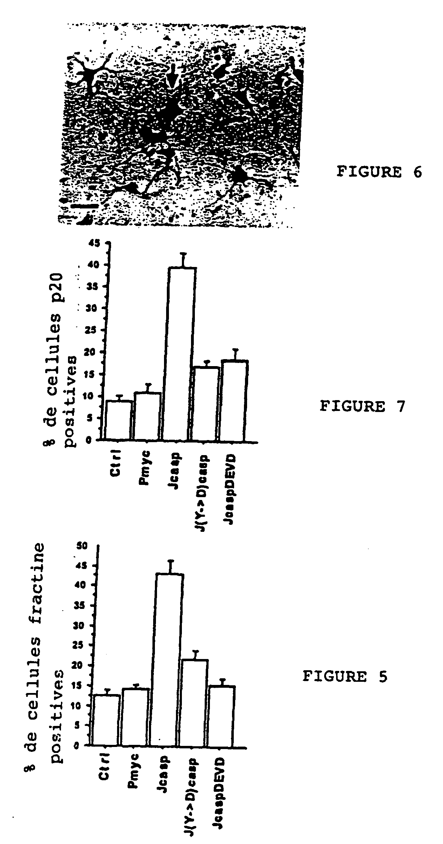

Les figures 4 et 5 illustrent la quantification du clivage de l'actine par la caspase 3 à l'aide de l'anticorps anti-fractine, par immunocytochimie après fixation des cellules au paraformaldéhyde (F. Yang et al., Am. J. Pathol., 1998, 152, 2, 379-389). L'anticorps anti-fractine reconnaît spécifiquement l'actine clivée par la caspase 3. Le pourcentage de neurones positifs pour la fractine a été déterminé après 24h d'internalisation des peptides, par comptage d'environ 1000 cellules par puits en triple. En présence des peptides G (KQYTSIHHG = SEQ ID NO:2 dans laquelle Y₁ représente un peptide d'internalisation et d'adressage, tel que défini ci-dessus et Y₀ est nul) (1X et 2X) et Gcasp (ou Jcasp) (KKKQYTSIHHGWEVD= SEQ ID NO:4 dans laquelle Y₁ représente un peptide d'internalisation et d'adressage, tel que défini ci-dessus et Y₀ VVEVD) (1X), il existe une augmentation significative du clivage par l'actine (G1X : p<0.0003 ; G2X : p<0.0001 ; Gcasp1X : p<0.0001). Le zVAD seul inhibe tout clivage endogène des neurones par les caspases et inhibe significativement l'augmentation de ce clivage par G1X, G2X et Gcasp1X (G1X/zVADG1X : p<0.0001 ; G2X/zVADG2X : p<0.0001; Gcasp/zVADGcasp : p<0.0001), même si l'inhibition n'est pas totale.

La figure 5 montre que le peptide Jcasp induit un clivage de l'actine. Elle montre également que le peptide J(Y→D)casp est peu actif et que l'inhibiteur zDEVD-fmk, plus spécifique de la caspase 3, inhibe le clivage de l'actine induit par le peptide Jcasp.

### - quantification du clivage de l'actine par la caspase 3 par mesure de la p20

Pour vérifier que la caspase 3 est effectivement impliquée dans l'apoptose provoquée par le peptide Jcasp, le fait que cette enzyme (caspase 3) est synthétisée sous la forme d'un propeptide (37 kDa), qui après stimulation, génère une sous-unité active de 17-22 kDa (p20) est utilisé.

L'immunoréactivité pour p20 est examinée dans des neurones embryonnaires corticaux de souris cultivés pendant 24 heures en présence de plusieurs peptides. La figure 6 illustre l'immunoréactivité de la protéine p20 et la figure 7 quantifie l'induction de la p20.

Le peptide Jcasp (2,4 µM) induit une maturation de la p20 ; on obtient un effet significativement moindre avec le peptide J(Y→D)casp, confirmant l'importance du résidu tyrosine dans l'induction de la caspase 3.

L'inhibiteur zDEVD-fmk décroît de manière significative l'activation de la p20, suggérant que l'apoptose induite par le peptide Jcasp implique la maturation de la caspase 3.

Les Inventeurs ont donc bien montré le caractère pro-apoptotique du peptide G internalisé grâce à sa liaison au vecteur V1.

Une telle propriété est d'intérêt pour les raisons suivantes :
1. Le domaine C-terminal entier n'est pas pro-apoptotique
2. II existe un site de clivage par les caspases entre les résidus aspartate (D) et alanine (A) marqués en gras dans la séquence de l'APP-Cter (figure 1).

On peut donc émettre l'hypothèse que le clivage entre D et A démasque une séquence KKKQYTSIHHGWEVD (= SEQ ID NO:4, dans laquelle Y₁ est nul est Y₀ représente VVEVD) à activité apoptotique. Ceci est particulièrement important car cela met en évidence un mécanisme impliqué dans la perte neuronale qui accompagne des démences de type Alzheimer.

Avoir identifié un peptide dérivé de l'APP correspondant à un domaine, normalement exposé après clivage *in vivo* et capable de provoquer l'entrée des cellules en apoptose, a comme premier avantage de proposer un mécanisme original susceptible d'éclairer certains aspects de la pathologie d'Alzheimer et donc de découvrir des voies thérapeutiques nouvelles (mise au point d'inhibiteurs).

Par ailleurs la liaison de la séquence G au vecteur V1 permet de fabriquer un peptide qui, une fois internalisé par les neurones en culture, provoque leur apoptose. Du fait des propriétés de V1, l'entrée se fait dans 100% des cellules quel que soit leur degré de maturation *in vitro* et ces cellules sont normales (cultures primaires).

L'aspartate en position 664 correspond au site de clivage de la caspase ; le peptide selon l'invention, lorsqu'il est internalisé dans des cellules neuronales, active la caspase 3, provoque le clivage de l'actine au niveau d'un site sensible à la caspase et induit la fragmentation de l'ADN.

De manière surprenante, les peptides conformes à l'invention, qui ne comprennent pas le BAP présentent ainsi des propriétés pro-apoptiques tant *in vitro* qu'*in vivo.*

### EXEMPLE 3 : Résultats in vivo

Les résultats *in vitro* (voir Exemple 2) démontrent que l'internalisation de Jcasp par les neurones corticaux de souris ou de rat provoque une apoptose par activation d'une caspase et suggèrent que la caspase 3 est l'une des caspases activées.

Pour vérifier si le peptide Jcasp est également actif *in vivo* et dans le cerveau adulte, 1 µl de peptide Jcasp, de peptide J(Y→D)casp (chacun à 2,7 µM dans un tampon salin) ou de tampon salin est injecté dans le cortex cérébral de souris. 24 heures plus tard, le nombre de cellules positives à la fractine est quantifié de chaque côté du site d'injection.

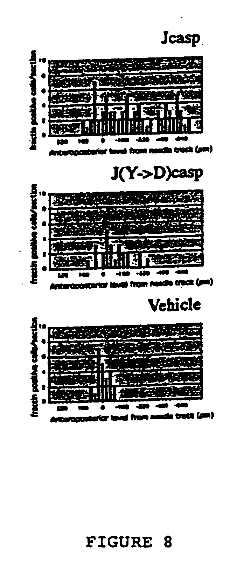

La figure 8 illustre les résultats obtenus. Bien que des variations aient été observées entre les différentes expériences, toutes donnent des résultats similaires montrant un effet important et spécifique du peptide Jcasp sur le nombre de cellules positives à la fractine (moyenne ± SEM : Jcasp (8 animaux), 40,7 ± 10,9 ; J(Y→D)casp (6 animaux), 13 ± 3,2 ; contrôle (3 animaux) : 8 ± 8 ; Jcasp vs contrôle : p<0,05 ; Jcasp vs J(Y→D)casp : p<0,04 ; contrôle vs J(Y→D)casp : NS).

Des applications *in vivo,* avec perfusion du peptide à l'aide de mini-pompes, sont aussi possibles.

A partir de là, ce système constitue un test rapide et simple pour cribler des bibliothèques de produits agissant spécifiquement sur la mort apoptotique induite par ce peptide et inoffensif sur d'autres modèles d'apoptose.

L'identification de telles substances est donc très utile pour la mise au point de traitements de l'apoptose accompagnant la maladie d'Alzheimer.

## Revendications

1. Peptide, **caractérisé en ce qu'**il est sélectionné dans le groupe des peptides définis par les séquences (code une lettre): Y₁KQYTSIHHGY₀ (SEQ ID NO:2), Y₁KKQYTSIHHGY₀ (SEQ ID NO:3) et Y₁KKKQYTSIHHGY₀ (SEQ ID NO:4), dans lesquelles Y₀ est nul ou représente V, VV, VVE VVEV ou VVEVD et Y₁ représente un peptide d'internalisation et d'adressage, répondant à la séquence X₁X₂X₃X₄X₅X₆X₇X₈X₉X₁₀X₁₁X₁₂X₁₃X₁₄X₁₅X₁₆, dans laquelle X₁, X₂, X₃, X₄, X₅, X₆, X₇, X₈, X₉, X₁₀, X₁₁, X₁₂, X₁₃, X₁₄, X₁₅, X₁₆ représentent chacun un acide α-aminé, 6 à 10 d'entre lesdits acides aminés étant hydrophobes et X₆ représentant un tryptophane.

2. Peptide selon la revendication 1, **caractérisés en ce que** la séquence Y₁ correspond à la séquence KQIKIWFQNRRMKWKK (SEQ ID NO:5).

3. Utilisation d'un peptide comprenant la séquence KKKQYTSIHHGVVEVD, pour la sélection et le criblage de produits aptes à inhiber l'apoptose.

4. Utilisation selon la revendication 3, **caractérisée en ce que** ledit peptide est associé à un peptide d'internalisation sélectionné dans le groupe constitué par les peptides d'internalisation aptes à passer la barrière hémato-encéphalique.

5. Utilisation d'un peptide sélectionné dans le groupe des peptides définis par les séquences (code une lettre) Y₁KQYTSIHHGY₀ (SEQ ID NO:2), Y₁KKQYTSIHHGY₀ (SEQ ID NO:3) et Y₁KKKQYTSIHHGY₀ (SEQ ID NO:4), dans lesquelles Y₀ est nul ou représente V, VV, VVE VVEV ou VVEVD et Y₁ est nul ou représente un peptide d'internalisation et d'adressage, répondant à la séquence X₁X₂X₃X₄X₅X₆X₇X₈X₉X₁₀X₁₁X₁₂X₁₃X₁₄X₁₅X₁₆, dans laquelle X₁, X₂, X₃, X₄, X₅, X₆, X_{7.} X₈, X₉, X₁₀, X₁₁, X₁₂, X₁₃, X₁₄, X₁₅, X₁₆ représentent chacun un acide α-aminé, 6 à 10 d'entre lesdits acides aminés étant hydrophobes et X₆ représentant un tryptophane, pour la sélection et le criblage de produits apte à inhiber l'apoptose.

6. Utilisation de cellules, dans lesquelles un peptide tel que défini aux revendications 3 à 5, a été intemalisé, pour la sélection et le criblage de produits aptes à inhiber l'apoptose.

7. Procédé de criblage et de sélection de produits aptes à inhiber l'apoptose, **caractérisé en ce qu'**il comprend :
- la mise en contact de l'inhibiteur potentiel avec une cellule dans laquelle un peptide tel que défini aux revendications 3 à 5, a été internalisé et
- la mesure du clivage de l'ADN ou de l'actine ou la mesure de la sous-unité p20 de la caspase 3.

8. Utilisation d'un peptide tel que défini aux revendications 3 à 5, pour la préparation d'un médicament anti-cancéreux.

9. Peptide, **caractérisé en ce qu'**il est sélectionné dans le groupe des peptides définis par les séquences (code une lettre) Y₁KQYTSIHHGY₀ (SEQ ID NO:2) et Y₁KKQYTSIHHGY₀ (SEQ ID NO:3), dans lesquelles Y₀ est nul ou représente V, VV, VVE VVEV ou VVEVD et Y₁ est nul, et par le peptide de formule Y₁KKKQYTSIHHGY₀ (SEQ ID NO:4), dans lequel Y₀ représente VVEVD et Y₁ est nul.

## Claims

1. A peptide, **characterized in that** it is selected from the group of peptides defined by the sequences (one-letter code): Y₁KQYTSIHHGY₀ (SEQ ID NO:2), Y₁KKQYTSIHHGY₀ (SEQ ID NO:3) and Y₁KKKQYTSIHHGY₀ (SEQ ID NO:4), in which Y₀ is null or represents V, W, WE, VVEV or WEVD and Y₁ represents an internalization and addressing peptide corresponding to the sequence X₁X₂X₃X₄XₛX₆X₇X₈X₉X₁₀X₁₁X₁₂X₁₃X₁₄X₁₅X₁₆, in which X₁ X₂, X₃, X₄, X₅, X₆, X₇, X₈, X₉, X₁₀, X₁₁, X₁₂, X₁₃, X₁₄, X₁₅ and X₁₆ each represent an α-amino acid, 6 to 10 of said amino acids being hydrophobic and X₆ representing a tryptophan.

2. The peptide as claimed in claim 1, **characterized in that** the sequence Y₁ corresponds to the sequence KQIKIWFQNRRMKWKK (SEQ ID NO:5).

3. The use of a peptide comprising the sequence KKKQYTSIHHGVVEVD, for selecting and screening products capable of inhibiting apoptosis.

4. The use as claimed in claim 3, **characterized in that** said peptide is combined with an internalization peptide selected from the group consisting of internalization peptides capable of crossing the blood-brain barrier.

5. The use of a peptide selected from the group of peptides defined by the sequences (one-letter code) Y₁KQYTSIHHGY₀ (SEQ ID NO:2), Y₁KKQYTSIHHGY₀ (SEQ ID NO:3) and Y₁KKKQYTSIHHGY₀ (SEQ ID NO:4), in which Y₀ is null or represents V, W, WE, VVEV or VVEVD and Y₁ is null or represents an internalization and addressing peptide corresponding to the sequence X₁X₂X₃X₄X₅X₆X₇X₈X₉X₁₀X₁₁X₁₂X₁₃X₁₄X₁₅X₁₆, in which X₁, X₂, X₃, X₄, X₅, X₆, X₇, X₈, X₉, X₁₀, X₁₁, X₁₂, X₁₃, X₁₄, X₁₅ and X₁₆ each represent an α-amino acid, 6 to 10 of said amino acids being hydrophobic and X₆ representing a tryptophan, for selecting and screening products capable of inhibiting apoptosis.

6. The use of cells, into which a peptide as defined in claims 3 to 5 has been internalized, for selecting and screening products capable of inhibiting apoptosis.

7. A method for screening and selecting products capable of inhibiting apoptosis, **characterized in that** it comprises:
- bringing the potential inhibitor into contact with a cell into which a peptide as defined in claims 3 to 5 has been internalized, and
- measuring cleavage of DNA or of actin or measuring the p20 subunit of caspase 3.

8. The use of a peptide as defined in claims 3 to 5, for preparing an anticancer medicinal product.

9. A peptide, **characterized in that** it is selected from the group of peptides defined by the sequences (one-letter code) Y₁KQYTSIHHGY₀ (SEQ ID NO:2) and Y₁KKQYTSIHHGY₀ (SEQ ID NO:3), in which Y₀ is null or represents V, VV, VVE, VVEV or VVEVD and Y₁ is null, and of the peptide of formula Y₁KKKQYTSIHHGY₀ (SEQ ID NO:4), in which Y₀ represents VVEVD and Y₁ is null.

## Patentansprüche

1. Peptid, **dadurch gekennzeichnet, dass** es aus der Gruppe von Peptiden ausgewählt ist, die durch die Sequenzen (Ein-Buchstabencode):
Y₁KQYTSIHHGY₀ (SEQ ID Nr.:2), Y₁KKQYTSIHHGY₀ (SEQ ID Nr.: 3) und
Y₁KKKQYTSIHHGY₀ (SEQ ID Nr.:4) definiert sind, wobei Y₀ Null ist oder V, VV, VVE, VVEV oder VVEVD wiedergibt und Y₁ ein Peptid zur Internalisierung und zur Vermittlung wiedergibt, das der Sequenz
X₁X₂X₃X₄X₅X₆X₇X₈X₉X₁₀X₁₁X₁₂X₁₃X₁₄X₁₅X₁₆ entspricht, in der X₁X₂X₃X₄X₅X₆X₇X₈X₉X₁₀X₁₁X₁₂X₁₃X₁₄X₁₅X₁₆ jeweils eine α-Aminosäure wiedergeben, 6 bis 10 der genannten Aminosäuren hydrophob sind und X₆ Tryptophan wiedergibt.

2. Peptid nach Anspruch 1, **dadurch gekennzeichnet, dass** die Sequenz Y₁ der Sequenz KQIKIWFQNRRMKWKK (SEQ ID Nr.: 5) entspricht.

3. Verwendung eines Peptids, umfassend die Sequenz KKKQYTSIHHGWEVD, zur Selektion und Klassierung von Produkten, die geeignet sind die Apoptose zu hemmen.

4. Verwendung nach Anspruch 3, **dadurch gekennzeichnet, dass** das genannte Peptid mit einem Peptid zur Internalisierung verbunden ist, dass aus der Gruppe von Peptiden zur Internalisierung ausgewählt ist, die geeignet sind die Blut-Hirn-Schranke zu überwinden.

5. Verwendung eines Peptids, ausgewählt aus einer Gruppe von Peptiden, die durch die Sequenzen (Ein-Buchstabencode) Y₁KQYTSIHHGY₀ (SEQ ID Nr.:2), Y₁KKQYTSIHHGY₀ (SEQ ID Nr.: 3) und Y₁KKKQYTSIHHGY₀ (SEQ ID Nr.:4) definiert sind, wobei Y₀ Null ist, oder V, VV, VVE, VVEV oder VVEVD wiedergibt und Y₁ Null ist oder ein Peptid zur Internalisierung und zur Vermittlung wiedergibt, das der Sequenz X₁X₂X₃X₄X₅X₆X₇X₈X₉X₁₀X₁₁X₁₂X₁₃X₁₄X₁₅X₁₆ entspricht, in der X₁X₂X₃X₄X₅X₆X₇X₈X₉X₁₀X₁₁X₁₂X₁₃X₁₄X₁₅X₁₆ jeweils eine α-Aminosäure wiedergeben, 6 bis 10 der genannten Aminosäuren hydrophob sind und X₆ Tryptophan wiedergibt, zur Selektion und Klassierung von Produkten, die geeignet sind die Apoptose zu hemmen.

6. Verwendung von Zellen, in denen ein Peptid, wie es nach den Ansprüchen 3 bis 5 definiert ist, internalisiert wurde, zur Selektion und Klassierung von Produkten, die geeignet sind die Apoptose zu hemmen.

7. Verfahren der Selektion und Klassierung von Produkten, die geeignet sind die Apoptose zu hemmen, **dadurch gekennzeichnet, dass** es umfasst:
- in Kontakt bringen des potentiellen Inhibitors mit einer Zelle, in der ein Peptid, wie es nach den Ansprüchen 3 bis 5 definiert ist, internalisiert wurde und
- Messung der DNA-Spaltung oder des Aktins oder Messung der p20-Untereinheiten der Caspase-3.

8. Verwendung eines Peptids, wie es nach den Ansprüchen 3 bis 5 definiert ist, zur Herstellung eines Anti-Krebs-Medikaments.

9. Peptid, **dadurch gekennzeichnet, dass** es aus der Gruppe von Peptiden ausgewählt ist, die durch die Sequenzen (Ein-Buchstabencode): Y₁KQYTSIHHGY₀ (SEQ ID Nr.:2), Y₁KKQYTSIHHGY₀ (SEQ ID Nr.: 3) definiert sind, wobei Y₀ Null ist, oder V,VV, VVE, VVEV wiedergibt und Y₁ Null ist, und durch ein Peptid der Formel Y₁KKKQYTSIHHGY₀ (SEQ ID Nr.:4), wobei Y₀ VVEVD wiedergibt und Y₁ Null ist.


### LISTE DE SEQUENCES

<110> CENTRE NATIONAL DE LA RECHERCHE SCIENTIFIQUE CNRS
   ALLINQUANT Bernadette
   PROCHIANTZ Alain
<120> NOUVELLES APPLICATIONS DE PEPTIDES ISSUS DU DOMAINE CYTOPLASMIQUE DU PRECURSEUR DE LA PROTEINE AMYLOIDE.
<130> BLOcp644PCT45
<140>
   <141>
<160> 9
<170> Patent In Ver. 2.1
<210> 1
   <211> 47
   <212> PRT
   <213> Homo sapiens
<400> 1
   Lys Lys Lys Gln Tyr Thr Ser Ile His His Gly Val Val Glu Val Asp
   1 5 10 15
   Ala Ala Val Thr Pro Glu Glu Arg His Leu Ser Lys Met Gln Gln Asn
   20 25 30
   Gly Tyr Glu Asn Pro Thr Tyr Lys Phe Phe Glu Gln Met Gln Asn
   35 40 45
<210> 2
   <211> 9
   <212> PRT
   <213> Homo sapiens
<400> 2
<210> 3
   <211> 10
   <212> PRT
   <213> Homo sapiens
<400> 3
<210> 4
   <211> 11
   <212> PRT
   <213> Homo sapiens
<400> 4
<210> 5
   <211> 16
   <212> PRT
   <213> Homo sapiens
<400> 5
   Lys Gln Ile Lys Ile Trp Phe Gln Asn Arg Arg Met Lys Trp Lys Lys
   1 5 10 15
<210> 6
   <211> 9
   <212> PRT
   <213> Homo sapiens
<400> 6
   Val Asp Ala Ala Val Thr Pro Glu Glu
   1 5
<210> 7
   <211> 9
   <212> PRT
   <213> Homo sapiens
<400> 7
   Asn Gly Tyr Glu Asn Pro Thr Tyr Lys
   1 5
<210> 8
   <211> 15
   <212> PRT
   <213> Homo sapiens
<400> 8
   Lys Lys Lys Gln Tyr Thr Ser Ile His His Gly Val Val Glu Val
   1 5 10 15
<210> 9
   <211> 7
   <212> PRT
   <213> Homo sapiens
<400> 9
   Gly Tyr Glu Asn Pro Thr Tyr
   1 5